**Europäisches Patentamt**

**European Patent Office**

**Office européen des brevets**

(19)

(11) Publication number:

**0 090 779**
**A2**

(12)

# EUROPEAN PATENT APPLICATION

(21) Application number: 83830072.1

(51) Int. Cl.³: **A 61 B 5/00**

(22) Date of filing: 31.03.83

(30) Priority: 31.03.82 IT 937282

(43) Date of publication of application: 05.10.83
Bulletin 83/40

(84) Designated Contracting States: **AT BE CH DE FR GB LI LU NL SE**

(71) Applicant: **Biocardio S.r.l., Località La Soterna, I-50032 Borgo San Lorenzo (Firenze) (IT)**

(72) Inventor: **Romagnoli, Pierluigi, Via Faentina 111, Ronta (Firenze) (IT)**
Inventor: **Lombardi, Paolo, Via Carducci 13, Firenze (IT)**
Inventor: **Pernice, Luigi Maria, Via Lungarno del Tempio 34/4, Firenze (IT)**
Inventor: **Andreoli, Francesco, Via Arnolfo 6/A, Firenze (AT)**

(74) Representative: **Bardini, Marco Luigi et al, c/o Società Italiana Brevetti S.p.A. Corso dei Tintori 25, I-50122 Firenze (IT)**

(54) **Method and apparatus for the execution of a gastro-esophageal pHmetric test.**

(57) An apparatus for the execution of a gastro-esophageal pHmetric test, particularly for pH monitoring throughout 24 hours, avoiding patient hospitalization and allowing the latter to continue normal life habits. It comprises a pHmetric electrode opportunely positioned in the patient's esophagus and/or stomach and a portable recording unit of very small dimensions, that is given to the patient to be tested, connected to said electrode. The recording unit comprises voltage-frequency converter that receives a DC signal from the electrode and produces a frequency signal proportional to the amplitude of the input signal; it also comprises a timer connected between said converter and a recording device, for enabling the frequency signal to be transferred to said device and simultaneously for actuating the same in correspondence with prefixed sampling intervals. The apparatus comprises finally a unit for analysing said frequency signal and visualising the test results.

BIOCARDIO S.r.l.

Method and apparatus for the ex cution

of a gastro-esophageal pHmetric test.

The invention refers to a method and an apparatus for the execution of gastro-esophageal pHmetric tests.

More precisely, the invention refers to an apparatus for carrying out the pHmetric gastro-esophageal monitoring throughout 24 hours with the aim to follow the variations of the intraluminal hydrogenionic concentration due to acid-peptic and/or alkalin refluxes.

Pathologic gastro-esophageal refluxes are verified through the insufficiency of the containing valves of the gastro-esophageal junction, related as is known, not so much to anatomic situations, as to functional factors, with clear predominance of a high pressure zone in correspondence with the lower esophageal sphincter (L.E.S.).

The reflux entity can be ascertained on the basis of precise parameters: quantity, duration, composition of

- 2 -

0090779

the reflux material and esophageal clearing time.

Within certain limits, (duration inferior to four minutes, total reflux duration less than ten minutes in the 24 hrs., absence of alkalin refluxes) the phenomenon is present in about 55% of healthy patients.

The specific event produces a duplicate order of effects when it exceeds the entity considered as normal: on one side, the esophageal mucuos producing a chemical esofagitis, on the other, causing a more or less marked alteration of the esophageal mobility with negative effect on the continence and the clearing capacity through the hypotensive action of the L.E.S.

The effects described, as appears evident, result mutually exalted, realizing a vicious circle that brings to a clinical state of esofagitis that becomes progressively more serious in variable time periods.

The pHmetering of the lower III of the esophagus, executed with the use of a miniaturized endoluminal electrode, represents the ideal method for the diagnosis of the acidpeptic and/or alkalin reflux. This method has been object in these years of rapid progress concerning, on one side, the quality of the instruments used and, on the other, the refining of the application modes.

The technical execution of the test consists in positioning an exploring electrode, connected to an amplifier-recording instrument, in the esophagus, 5 cm above the L.E.S.. The test must be preceded by a manometric exam, that permits, besides, to define the seat and pressure of the L.E.S.

The above described method was originally used for samplings of brief duration; to this purpose we recall the

- 3 -

0090779

test proposed by Tuttle and Grossman called "pull-through", the "reflux test" of Hill and Coll, the "acid clearing test" of Booth and Bernstein's test, which only analyze partial aspects of the reflux physiopathology. The above described tests are still used in a combined manner for the so called "brief pHmonitoring". This method, even if it corresponds without doubt to an economic utility, has been demonstrated as inadequate for the specific aims of the test, as it introduces numerous factors of error depending, in synthesis, on the artificial acidification of the stomach and on the execution of conventional operations (epigastric compression, assumption of Tredelemburg's position, Valsalva and Muller's position, etc.) thus resulting affected by a very high incidence of errors.

Modernly, this method is almost abandoned, instead the esophageal pHmeter monitoring of the 24 hours is used, test that now is identified unaninmously with the name of esophageal pHmetering. The prolonged recording of the pH permits to follow variations of the intraluminal hydrogenionic concentration during an arc of time and eliminates artificial interventions on the patient. The application of this method generally foresees that the subject under examination is connected to the recording instruments for the whole duration of the monitoring, executed in hospital. The patient, even though free to pursue limited activities, results per force impeded by the length of the connecting cables, usually not longer than 2,5 m. It is obvious how in such a manner the patient's habits are radically changed and the 24 hours of the test do not reflect, not even summarily, the patient's normal way of life.

It is an object of the present invention to provide an apparatus for the execution of pHmetric gastro-esophageal tests and in particular, for the monitoring throughout

0090779

24 hours, that, after the positioning of the sampling electrode in the esophagus and/or stomach of the patient, can be given to the latter without creating the least problem of hospitalization and incumbrance, allowing the patient to pursue normal daily activities and consequently, increasing the degree of reliability of the pHmetric test's results.

Another object of this invention is to provide a portable apparatus for the recording of direct current signals produced by an electrode, in particular, a pHmetric electrode, said apparatus being also of reduced dimensions and low cost.

A further object of this invention, is to provide an apparatus that enables to read, decode and visualise the data sampled and recorded by a pHmetric electrode positioned in the patient's esophagus and/or stomach.

A further object of this invention is to provide a method of recording and reading direct current signals coming from a pHmetric electrode positioned in the patient's esophagus and/or stomach.

These objects are reached by providing an apparatus for the execution of gastro-esophageal pHmetric tests and in particular, the monitoring throughout 24 hours, comprising a pHmetric electrode, suitably positioned in the patient's esophagus and/or stomach and a portable recording unit, to which said electrode is connected, for the recording of direct current signals coming from it. This unit comprises:

- voltage-frequency converting means for receiving a D.C. input signal and for producing an output signal having a frequency directly proportional to the amplitude of the input signal;

- a recording device connected to the output of said voltage-frequency converting means;

- a timer connected to said converting means and to the recording device for enabling, in correspondence with prefixed sampling intervals, respectively the transfer of the frequency signal to the recording device and the activation of said device.

In a practical embodiment of the invention the recording device that is given to the patient, is a battery operated tape recorder, of very reduced dimensions (15 x 10 x 3,5 cm) and very light (650 g). This recording device is equipped with an event marker at the patient's disposal and can be equipped with an auto-reverse system. It uses a commercial compact-cassette of 45' per side. In order to coincide the total duration of the tape with the total duration of the test (24 hours), the apparatus is able to execute a sampling of the continuous signal emitted by the electrode, sampling which consists in executing the recording of the frequency-converted signal at prefixed rate and with a tape advancement (for example at the speed of 4,75 cm/s) that occurs at intervals corresponding exactly to the sampling rate.

Suitably setting the sampling ratio, i.e. the ratio between the rate and duration of the recording, it is possible to effect a continuous recording of 24 hours on one sole tape.

The reading of this recording is performed by a plotter that decodifies the signal and shows on a digital display of any known type, on request of the operator, the number of acid and/or alkalin refluxes, the number of acid refluxes with a duration superior to 4 minutes (those that are considered as pathological), the duration of each single reflux episode, the eventual con-
comittance with a symptomatology, signalled by the patient
through the event marker, as well as the total and percent reflux time.   The parameters described can refer
both to partial timings and to the entire duration of
the 24 hours.

In the same manner it is also possible to obtain a layout of the pH monitoring.

The advantages arising from the use of the apparatus
according to the invention are apparent.   First of all,
there  exists a precise correspondence between the
patient's normal life habits and what occurs       during
pH monitoring.   Besides, hospitalization becomes unnecessary, obtaining consequently both a decrease in
social costs, as well as a major tolerance of the test
for the patient.   The degree of  reliability of the
pHmetric evaluation is greatly increased both for the
above mentioned reasons and also in view of the fact
that the data are entirely computerized, thus eliminating any reading subjectiveness.   It is also possible to use the testing method connected to the apparatus
according to the present invention in non-specialized
hospital departments, thanks to the microprocessor
technology.   Finally, a further important advantage of
the present apparatus is the very low cost thereof with
respect to its performance and quality of the results,
in comparison with the tecniques already known and adopted up to now.

A preferred embodiment of the apparatus according to the
invention  will now be described in more detail, by way
of a not limiting example, with reference to the attached
drawings in which:

- figure 1 shows a block diagram of the portable recording unit;

- figure 2 shows a block diagram of a computerized analysing decodifying unit;

- figure 3 illustrates the circuitry corresponding to the block diagram of figure 1;

- figure 4 illustrates the circuitry corresponding to the block diagram of figure 2 excluding the computerized part.

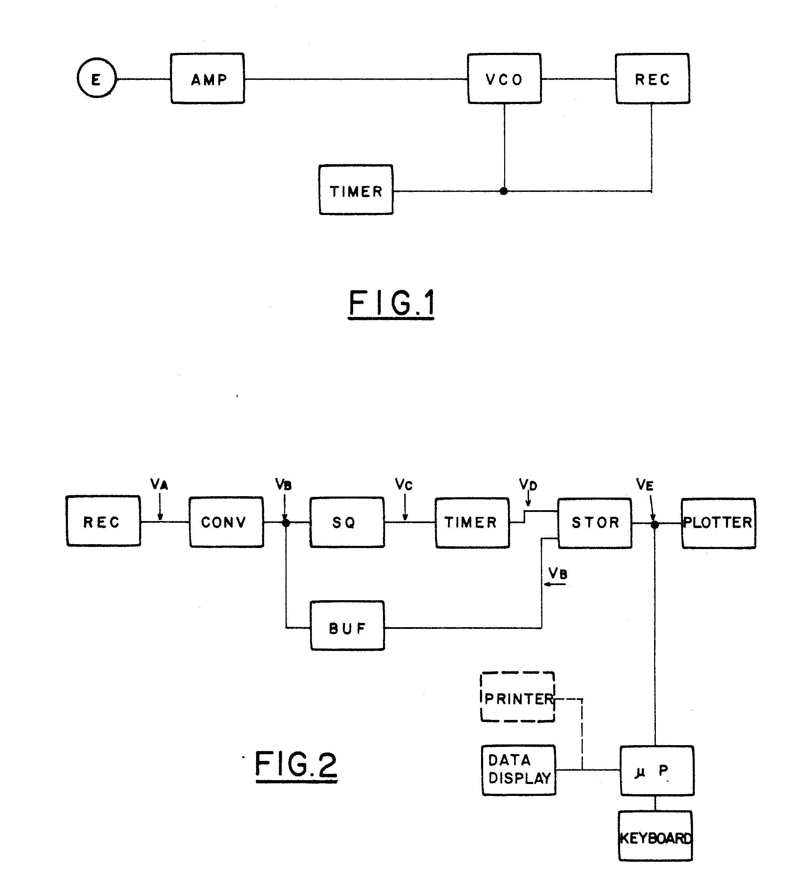

With reference to figure 1, an esophageal electrode E for pHmetric samplings of known type, for example a calomelano electrode, positioned in the patient's esophagus, sends out signals, practically in direct current and at a very high impedance, to an input amplifier that turns them into low impedance signals and amplifies them.  The D.C. signal treated in this way is converted into a square wave with a duty cycle equal to 50% through a voltage-controlled oscillator VCO.  The frequency signal is tape-recorded by the part of cicuitry, of the conventional type, indicated by REC.  The recording  is not carried out in a continuous manner, but since a long duration (24 hours) recording has to be performed using a common C 90 cassette with a  real time autonomy of 90 minutes, a system has been adopted that at a prefixed rate permits access to REC of the frequency to be recorded.  Using for example a sampling ratio as defined above, approximately equal to 16, i.e. one second time of recording and 15 seconds of pause, as the signals sent by the electrode are extremely slow, the above mentioned sampling rate is more than sufficient to avoid missing any information.  The access to REC of the frequency signal from VCO, is enabled at the prefixed rate, by the timer, indicated as TIMER, that simultaneously actuates the tape  advancement.

Once the entire cassette has been recorded, its content

is anal₁sed by the unit schematically shown in figure 2. The playing of the tape is effected at a nominal speed of 4,75 cm per second.  The recorded frequencies are sent under form of a pulse train to a frequency-voltage converter, indicated by CONV, that cosequently operates inversely with respect to VCO of figure 1, i.e. it re-converts in direct current the frequency-recorded signal. Due to the impulsive advancement during the recording phase and to the inevitable mechanical inertiae, typical of the motor-tape system, between one recording and the successive one dead zones are produced, in which no signals are recorded, so in the audition phase, the pulse trains relative to each sampling, are separated by an interval of time equal to a  few hundredths of second. This separation causes a fall in the voltage amplitude in output from CONV, called $V_B$, as illustrated in the following diagrams:

$V_A$ = input voltage

$f_1 > f_2 > f_3$
$V_1 \equiv f_1 \quad V_2 \equiv f_2 \quad V_3 \equiv f_3$
$T = t_2 - t_1 = t_4 - t_3 = t_6 - t_5$

$V_B$ = output voltage

It is apparent that every single pulse train is converted into a constant continuous voltage for the whole duration of the pulse train, except for an overshooting at the beginning of each conversion.  To execute correctly the treatment of this signal, both the initial overshooting and the final fall of amplitude, must be eliminated.  This has been realized using an analogic memory circuit, indicated by STOR, that reads the signal in the stable zone and maintains this value until the successive reading.

STOR reading control signal is constituted by the same signal $V_B$ to be read: this in fact is squared by SQ and the trailing edge of the square wave $V_C$, obtained in this way, generates through a double timer (TIMER), a delayed pulse $V_D$ having a delay $\tau_1$ with respect to the edge itself and a duration $\tau_2$ such as to finish before the end of the square wave of duration T. The wave-forms are shown here below:

These pulse readings $V_D$ are sent to an input of the memory STOR, while the voltage $V_B$, to be read, duly buffered by BUF, is sent to the other input.   As a result, according to the proposed example, a step-type output voltage $V_E$ is obtained from the memory, as previously shown.   This is the electric signal that is visualized and analysed by the computerized system that can be of traditional type.

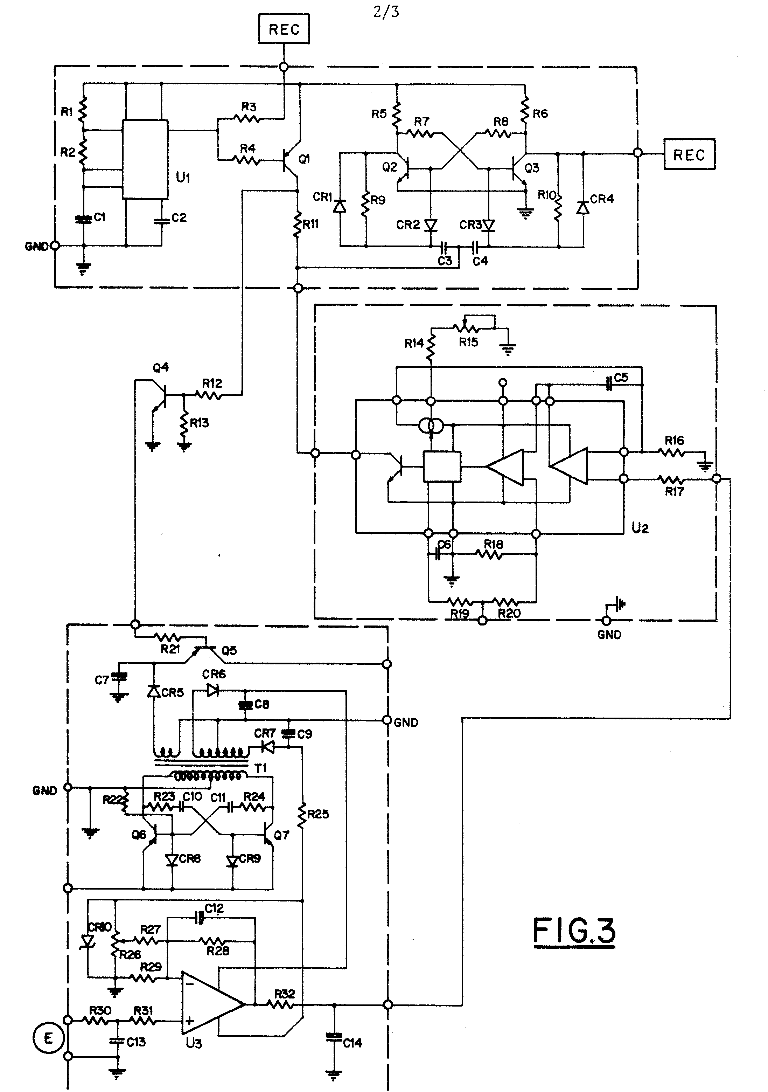

In figure 3 a possible circuit for the realization of the block diagram of figure 1, relevant to the portable unit for recording the signal from the electrode, is shown in detail. This signal is sent to the non-inverting input of an amplifier $U_3$ through the T network constituted by $R_{30}$, $C_{13}$, $R_{31}$.   A reference voltage, obtained from $CR_{10}$, $R_{26}$ and $R_{27}$, is sent to the inverting input of the same amplifier.   The gain of $U_3$ is established by $R_{28}$, $R_{29}$ and $C_{12}$ for an output voltage corresponding to 500 mv/˙pH. The supply  voltages of $U_3$ and $U_2$ are generated by a converter formed by $Q_6$, $Q_7$, $CR_8$, $CR_9$, $R_{22}$, $R_{23}$, $R_{24}$, $C_{10}$, $C_{11}$, $T_1$, followed by a rectifying and filtering section $CR_5$, $CR_6$, $CR_7$, $C_7$, $C_8$, $C_9$.

The output signal of $U_3$, filtered again by $R_{32}$, $C_{14}$, is sent to the voltage-frequency converter ($R_{14}$, $R_{15}$, $R_{16}$, $R_{17}$, $R_{18}$, $R_{19}$, $R_{20}$ and $C_6$) able to produce a frequency equal to 2KHz per volt of input voltage and  eacn time that $U_2$ is energized through $Q_1$, $Q_4$, $Q_5$, all actuated by $U_1$ that, with its network $R_1$, $R_2$, $C_1$ consitutes the timer of the whole system.

Consequently the flip-flop formed by $Q_2$, $Q_3$, $R_5$, $R_6$, $R_7$, $R_8$, $R_9$, $R_{10}$, $CR_1$, $CR_2$, $CR_3$, $CR_4$, $C_3$, $C_4$ is driven by $U_2$, only  when the last is energized.   This divides by two the frequency and generates a square wave signal with 50% of duty cycle and with a frequency equal to 1 KHz per volt of $U_3$ output voltage.   This is the signal that is sent to the tape-recording.   The timer constituted by $U_1$ through $R_3$ and $Q_9$ (not shown in figure 3) also energizes pulsively the tape advancing motor.

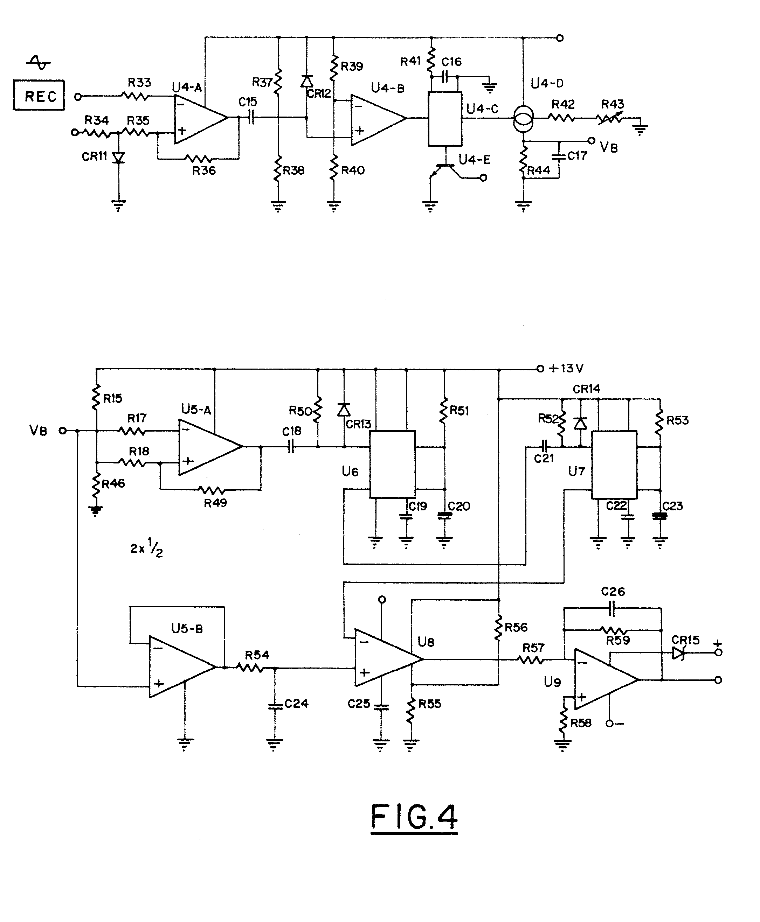

Once recording has been completed, the recorder is connected to the analysing unit and set in the listening position.   In this way the frequencies recorded are sent to a frequency-voltage converter, whose detailed circuitry diagram is shown in figure 4.  The recorded signal is squared by $U_{4A}$, $R_{33}$, $4_{34}$, $R_{35}$, $R_{36}$, $CR_{11}$, and derived by $C_{15}$, $R_{37}$, $R_{38}$, $R_{39}$, $R_{40}$, $CR_{12}$, $U_{4B}$ before being sent to a monostable $U_{4C}$, $R_{41}$, $C_{16}$ that generates a pulse of constant duration for each  recorded frequency period.   These pulses permit the constant current generator $U_{4D}$, $R_{42}$, $R_{43}$, to integrate its own current on $R_{44}$, $C_{17}$, until it obtains the voltage $V_B$.   This voltage is squared, with a phase inversion, by $U_{5A}$, $R_{45}$, $R_{46}$, $R_{47}$, $R_{48}$, $R_{49}$, derived by $C_{18}$, $R_{50}$, $CR_{13}$ and sent to $U_6$, $R_{51}$, $C_{19}$, $C_{20}$, that generate a pulse of duration $\tau_1 = 1.1 \cdot R_{51} \cdot C_{20}$, which, derived by $C_{21}$, $R_{52}$, $C_{14}$, drives a second monostable $U_7$, $R_{53}$, $C_{22}$, $C_{23}$.   This monostable sends a pulse of duration $\tau_2 = 1.1 \cdot R_{53} \cdot C_{23}$ to the "sample and hold" $U_8$, $R_{55}$, $R_{56}$, $C_{25}$, to which the voltage $V_B$, buffered by $U_{5B}$, is constantly sent and integrated by $R_{54}$, $C_{24}$.   The output of $U_8$ is impedance matched by $U_9$, $R_{57}$, $R_{58}$, $R_{59}$, and filtered by $C_{26}$.   This voltage is sent to a printing unit and/or an analyser computer unit.

According to this invention, a procedure for the recording and reading of a DC signal is proposed, this procedure comprising the phases of:

1 - sampling the value of the continuous voltage through the recording of a signal, having a frequency  proportional to the value of said continuous voltage, only in correspondence to prefixed sampling intervals.

2 - reading  the frequency-recorded signal and reconvert-

0090779

ing it in a voltage signal.

3 - producing a step voltage signal, the voltage of which remains constant and proportional to the value of the original continuous voltage for the whole duration of the sampling interval.

This invention is not to be considered as limited by the embodiment described herein and it is understood that it comprises any form of variation or modification which falls within the scope of invention itself.

CLAIMS

1. Apparatus for the execution of gastro-esophageal pHmetric tests and in particular, for the continuous pH monitoring throughout 24 hours, characterized by the fact that it comprises a pHmetric electrode to be suitably positioned in the esophagus and/or stomach of the patient, and a portable unit, to which said electrode is connected, for recording DC signals produced by said electrode, said unit comprising:

- voltage-frequency converting means for receiving a DC input signal and for producing an output signal having a frequency directly proportional to the amplitude of said input signal;

- a recording device connected to the output of said voltage-frequency converting means;

- a timer connected to said converting means and to said recording device for enabling, in correspondence with prefixed sampling intervals, respectively the transfer of frequency signals to the recording device and the activation of said recording device.

2. Apparatus according to claim 1, wherein an high-input-impedence amplifier connected between the source of said DC signal and the voltage-frequency converting means is provided.

3. Apparatus according to claim 2, wherein said amplifier comprises an operational amplifier, said electrode being connected to the non-inverting input thereof, the other input being connected to a continuous reference voltage.

4. Apparatus according to the previous claim, wherein a converter is provided comprising a generator of pulses

0090779

having a frequency twice the frequency required for the particular level of continuous voltage, connected to a bistable circuit that halves said frequency and generates a substantially simmetrical square wave, said timer energizing respectively the converter and the advancement motor of the recording device, only for a fraction of the cycle time given for each sampling.

5. Apparatus for the execution of gastro-esophageal pHmetric tests according to the previous claims, characterized by the fact that it includes a recorded signal reading unit comprising:

- frequency-voltage converting means, to be connected to the output of the recording device, for generating a continuous voltage signal proportional to the input signal;

- a memory circuit that stores the value of the voltage signal produced by said frequency-voltage converting means to which it is connected through an interface unit;

- a timer connected between said frequency-voltage converting means and said memory circuit for enabling the latter to start memorizing in correspondence to a prefixed interval of time and inside the sampling interval.

6. Apparatus according to claim 5 wherein the timer receives the signal recovered under form of pulses of prefixed duration and amplitude proportional to the frequency of the signal recorded and generates a sampling pulse of prefixed duration, but lower than one of the recovered signal, said sampling pulse being momentaneously contained inside the recovered pulse and obtained from the latter.

- 3 -

0090779

7. Apparatus according to claim 6, wherein said timer comprises a squaring circuit connected to a delaying circuit that is connected to a monostable pulse generator to avoid the sampling pulse be affected by initial and final transient state of the recovered signal.

8. Apparatus according to claim 7, wherein the memory circuit comprises a "sample and hold" operational amplifier that receives respectively the recovered voltage signal and the sampling pulse and maintains its output at a constant voltage value for the whole duration of the recovered pulse.

9. Procedure for recording and reading a DC signal, particularly a DC signal coming from a pHmetric electrode in a gastro-esophageal pHmetric test, characterized by the fact that it comprises the following steps:

- sampling the value of the continuous voltage and recording a signal having a frequency proportional to the value of said continuous voltage only in correspondence of prefixed sampling intervals;

- reading the frequency-recorded signal and reconverting it in a voltage signal;

- producing a step voltage signal, the voltage of which remains constant and proportional to the value of the original continuous voltage for the whole duration of the sampling interval.

0090779

## FIG.1

## FIG.2

FIG.3

FIG.4